# EUROPÄISCHE PATENTSCHRIFT

(11) **EP 1 305 041 B1**
(45) Veröffentlichungstag und Bekanntmachung des Hinweises auf die Patenterteilung: **21.06.2006**
(21) Anmeldenummer: 01958002.6
(22) Anmeldetag: 21.07.2001
(51) Int. Cl.: A61K 39/00, A61P 35/00, A61P 37/04, C12N 5/08

(54) **ARZNEIMITTEL ZUR IMMUNTHERAPIE MALIGNER TUMOREN**
MEDICAMENT FOR THE IMMUNOTHERAPY OF MALIGNANT TUMOURS
MEDICAMENT DESTINE A L'IMMUNOTHERAPIE DE TUMEURS MALIGNES

(30) Priorität: 28.07.2000 EP 00116362
(43) Veröffentlichungstag der Anmeldung: 02.05.2003
(73) Patentinhaber: LipoNova AG, 30625 Hannover (DE)
(72) Erfinder: ULBRICH, Claudia, 30419 Hannover (DE); ROCKENSÜSS, Klaus-Dieter, 34560 Fritzlar (DE); GROSSMANN, Armin, 30419 Hannover (DE)
(74) Vertreter: Meyers, Hans-Wilhelm
(86) Internationale Anmeldenummer: PCT/EP2001/008455
(87) Internationale Veröffentlichungsnummer: WO 2002/009745

(56) Entgegenhaltungen:
- WO-A-99/47687
- WO-A-99/51257
- DE-A- 19 633 731
- US-A- 6 077 519
- GROSSMANN A ET AL: "Active-specific immunotherapy of pancreatic carcinoma: Usefulness of human pancreatic carcinomas in preparing autologous tumor vaccines." ANTICANCER RESEARCH, Bd. 17, Nr. 4B, Juli 1997 (1997-07) - August 1997 (1997-08), Seiten 3117-3120, XP000990188 in der Anmeldung erwähnt

## Beschreibung

Gegenstand der vorliegenden Anmeldungen sind Zusammensetzungen, die sich insbesondere zur Immuntherapie maligner Tumoren eignen, sowie Verfahren zu deren Herstellung und die Verwendung der Zusammensetzungen zur Herstellung von Arzneimitteln.

Üblicherweise erfolgt die therapeutische Behandlung von Tumoren durch radikale Chirurgie, Chemo-, Radio- oder Hormontherapie. Diese Therapien haben zahlreiche unerwünschte Nebeneffekte und bringen erhebliche Belastungen für den Patienten mit sich. Überdies werden bei einigen Tumorformen mit diesen Therapien fast keine Verbesserungen erzielt, so dass deren Anwendung in Anbetracht der Nebenwirkungen nicht sinnvoll erscheint. Dazu zählen insbesondere maligne Tumoren, maligne Melanome, Nierenkarzinome, Darmkarzinome und pankreatische Karzinome. Daher liegt z. B. bei Nierenkarzinomen eine Sterblichkeitsrate von 85 % vor.

In den letzten-Jahren wurden verstärkt Erkenntnisse über das komplexe Zusammenspiel zwischen Tumoren und dem Immunsystem gewonnen. Dabei rückten Strategien zur Behandlung von Tumoren in den Mittelpunkt des Interesses, bei denen das Immunsystem stimuliert wird. Im Allgemeinen ist es das Ziel solcher Therapien, zu erreichen, dass das Immunsystem spezifische Antigene von Tumorzellen erkennt, die bei gesunden Zellen nicht oder in geringeren Mengen vorhanden sind. Dies wird z. B. durch die Applikation eines Arzneimittels erreicht, wie es in Anticancer Research [(1997) Nr. 17, Seiten 2879 - 2882 und 3117 - 3120] beschrieben wird : Dabei wird einem Patienten Tumorgewebe entnommen und zu einem autologen Tumorzellenlysat verarbeitet, welches dem Patienten injiziert wird. Dies geschah in der Erwartung, dass gegen die Tumorantigene im Lysat Immunität entsteht. Eine andere Strategie wird in der veröffentlichten Patentanmeldung WOA99/47687 beschrieben. Dort wird offenbart, dass Patienten autologe-Antigen präsentierende Zellen injiziert werden, die an ihren Oberflächen eine spezielle Tumordeterminante exprimieren.

Das Ziel von Tumortherapien ist es nicht nur, die Vergrößerung von Tumoren und die Bildung von Metastasen zu verhindern, sondern auch deren Rückbildung zu fördern. Die Lebenserwartung des Patienten soll erhöht und dessen Gesundheit und Lebensqualität verbessert werden. Über den Erfolg von Immuntherapien lässt sich zum jetzigen Zeitpunkt feststellen, dass die bisherigen therapeutischen Behandlungen leider nur Einzelerfolge oder Teilerfolge erzielen können. Ein grundlegendes Problem ist dabei, dass viele Tumormarker in gewissen Differenzierungsstadien und in gewissen Mengen auch in gesunden Zellen vorhanden sind. Daher erfolgt die Aktivierung des Immunsystems gegen solche Tumormarker oft nicht in dem gewünschten Ausmaß oder mit der erforderlichen Spezifität.

Aufgabe der vorliegenden Erfindung war es, Arzneimittel zur Tumortherapie zu entwickeln, die die oben genannten Ziele in hohem Maße erreichen. Tumortherapien sollten bei Verwendung der erfindungsgemäßen Arzneimittel auch relativ schnell und einfach durchführbar sein.

Die vorliegende Erfindung betrifft eine Zusammensetzung zur Immuntherapie von Tumoren. Die Zusammensetzung ist durch ein Verfahren erhältlich, in dem Tumormaterial bonitiert, zerkleinert und in eine gereinigte Zellsuspension überführt wird, die mit Interferon-gamma und Tocopherolacetat inkubiert und eingefroren wird, wodurch ein Tumorzelllysat entsteht, und in dem Monozyten aus buffy coats oder aus Vollblut isoliert werden, die anschließend durch Inkubation mit Zytokinen zur Differenzierung zu dendritschen Zellen angeregt und in das nichtadhärente Stadium überführt werden, wonach eine berechnete Menge des obigen eingefrorenen Tumorzelllysats aufgetaut wird, als Antigen zugesetzt wird, Zytokine zugesetzt werden, inkubiert wird und die entstandenen reifen dendritischen Zellen geerntet werden.

Unter der Bonitierung des Tumormaterials versteht man die makroskopische Beurteilung des Gewebes, nach der deutlich erkennbare Anteile an Fett-, Binde- und funktionalem Nierengewebe, Blutgefäße sowie weitere nicht-Tumorgewebe identifiziert und in der Folge entfernt und verworfen werden.

In einer besonderen Ausführungsform wird bei der Erzeugung der Zusammensetzung autologes Tumormaterial verwendet. Bei der Erzeugung der Zusammensetzung werden zur Differenzierung zu unreifen dendritischen Zellen bevorzugt IL-4 und GM-CSF und/oder IFN-gamma zugesetzt.

Die erfindungsgemäße Zusammensetzung eignet sich insbesondere als Arzneimittel oder zur Herstellung eines Arzneimittels zur Immuntherapie. Arzneimittel, die das erfindungsgemäße Zelllysat beinhalten, werden bevorzugt intrakutan oder subkutan injiziert.

Mit dem erfindungsgemäßen Arzneimittel können alle denkbaren Arten von soliden Tumorerkrankungen behandelt werden. Insbesondere eignen sich Arzneimittel, die die erfindungsgemäße Zusammensetzung beinhalten, zur Behandlung von Tumoren, bei denen andere Behandlungsmethoden wenig erfolgreich sind. Dies sind insbesondere neben anderen malignen soliden Tumoren maligne Melanome, Nierenkarzinome, Darmkarzinome, pankreatische Karzinome, Lymphome, Bronchialkarzinome und gynäkologische Tumoren.

Bei der Behandlung von Patienten mit dem erfindungsgemäßen Arzneimittel im Rahmen einer Tumortherapie wurden unerwartet ausgeprägte positive Effekte für die Patienten festgestellt. In überraschendem Ausmaße konnte das Wachstum von Tumoren und die Metastasenbildung verhindert werden, während die Rückbildung von Tumoren gefördert wurde. Die Gesundheit, die Lebensqualität und die Lebenserwartung der Patienten wurde deutlich erhöht. Wahrscheinlich können diese Effekte erzielt werden, weil sich das erfindungsgemäße Arzneimittel in wesentlichen Aspekten von den bekannten unterscheidet. Ein besonderer Unterschied zu vielen bekannten Verfahren ist, dass dem Patienten nicht einfach Tumormarker appliziert werden, sondern dass diese in dendritischen Zellen als Vehikel direkt in das Immunsystem des Patienten eingeschleust werden. Überraschenderweise genügt es dabei, den dendritischen Zellen *in vitro* ein krudes Zelllysat aus Tumorzellen zuzuführen, während nach WOA99/47687 antigenpräsentierende Zellen mit einem gereinigten Antigen versetzt oder transfiziert werden. Das erfindungsgemäße Verfahren ist daher auch schneller und einfacher durchführbar als bekannte Verfahren. Dies ist bei der Herstellung solcher Therapiestoffe von besonderer Bedeutung, damit die Gefahr von Kontaminationen gering gehalten wird. Des weiteren hat das Zelllysat den Vorteil, dass das gesamte Antigenrepertoire einer Tumorzelle verfügbar ist.

Die vorliegende Erfindung betrifft auch Verfahren zur Herstellung eines Arzneimittels, bei denen eine Suspension von Tumorzellen hergestellt wird, die Tumorzellen abgetötet werden und bei dem Monozyten aus Blut isoliert werden, deren Differenzierung zu dendritischen Zellen induziert wird und die so gewonnenen "unreifen" dendritischen Zellen mit dem Zelllysat der abgetöteten Tumorzellen inkubiert werden, die Reifung der dendritischen Zellen induziert wird und die "reifen" dendritischen Zellen geerntet werden.

Die Monozyten werden dabei bevorzugt aus buffy coats, aus Stammzellseparation, aus Leukaphereseprodukten oder aus Vollblut isoliert. Die Differenzierung der Monozyten zu "unreifen" dendritischen Zellen wird bevorzugt durch Zytokine, IL-4 und GM-CSF induziert. Zur Induktion der Reifung von "unreifen" zu "reifen" dendritischen Zellen eignen sich insbesondere Postaglandin E2 und TNF-α und/oder IL-1β und IL-6 zusätzlich zu IL-4 und GM-CSF. Die Herstellung der Tumorzellensuspension erfolgt im Allgemeinen durch Isolierung von Tumormaterial, welches gegebenenfalls bonitiert, zerkleinert und in eine gereinigte Zellsuspension überführt wird. In einer besonderen Ausführungsform des erfindungsgemäßen Verfahrens wird die Suspension von Tumorzellen aus autologem Tumormaterial hergestellt. In einer weiteren bevorzugten Ausführungsform wird in der Tumorzellensuspension vor dem Abtöten der Tumorzellen die Expression von membranständigen Proteinkomplexen induziert. Die Induktion erfolgt bevorzugt durch Interferon-gamma und Tocopherolacetat. Das Abtöten der Tumorzellen erfolgt insbesondere durch Einfrieren. Das Ernten der reifen dendritischen Zellen erfolgt bevorzugt bei Vorliegen typischer morphologischer Merkmale (z. B. Segelbildung), beurteilt durch mikroskopische Kontrolle und/oder durch Charakterisierung von Oberflächenantigenen mittels fluoreszierenden Antikörpern. Inhalt der Erfindung ist auch die Verwendung der beschriebenen Zusammensetzung und ihre möglichen Ausführungsformen zur Herstellung von Arzneimitteln zur Tumortherapie.

Die beschriebene Zusammensetzung und ihre möglichen Ausführungsformen werden erfindungsgemäß auch zur Herstellung von Arzneimitteln zur Tumorimpfung verwendet.

### Ausführungsbeispiel

### Herstellung einer Zusammensetzung zur Tumortherapie

### A) Herstellung eines Tumorzelllysats

Zur Präparation des Tumorgewebes werden makroskopisch deutlich erkennbare Anteile an nicht-Tumorgewebe wie Fett-, Binde- und funktionale Nierengewebe sowie Blutgefäße und nekrotisches Gewebe sorgsam entfernt und verworfen. Das fertig präparierte Gewebe wird so klein wie möglich zerteilt (Stückchen mit etwa 2-3 mm Durchmesser) und/oder herausgeschält und anschließend mit dem umgebenden Medium in ein steriles Sieb (50-100 mesh) überführt. Mit dem Glasstab werden alle im Sieb befindlichen Gewebestückchen unter langsamen Rühren ohne Druck durchpassiert. Die durchgetretenen Zellen werden mit dem Medium (RPMI 1640) in ein steriles Becherglas überführt und die Gewebereste im Sieb erneut nach Zugabe von 15 ml RPMI-Medium (RPMI 1640 mit 25 mmol HEPES) mit dem Glasstab passiert.

Die Zellsuspension wird auf 45% Percollkissen aufgeschichtet. Dieser Schritt dient zur Entfernung eventuell vorhandener Erythrozyten und zur Anreicherung von mononukleären Zellen auf dem Percollkissen. Die befüllten Röhrchen werden zentrifugiert, und die Interphase mit den mononukleären Zellen wird abgesaugt, in ein Röhrchen überführt, abzentrifugiert und mit NaCL-/Glucoselösung gewaschen. Die Gesamtzahl vitaler Zellen wird mikroskopisch mit Hilfe einer Neubauer-Zählkammer nach Färbung der Zellen mit Trypanblau bestimmt. Außerdem wird eine Zelltypisierung durchgeführt mit Testsimplets® (Boehringer, Mannheim), die sich eignen, Karzinomzellen in einem schnellen Färbeverfahren von anderen Zellen unterscheidbar zu machen. Nach Resuspension der Zellen in Natriumchlorid/Glucoselösung wird Vitamin E (700 µg/herzustellender Dosis) und Interferon-gamma (1500 I.E./herzustellender Dosis) zugegeben. Der Ansatz wird im Wasserbad zwei Stunden bei 37°C inkubiert, zentrifugiert und zweifach mit Natriumchlorid/Glucoselösung gewaschen. Der Ansatz wird in Kryoröhrchen aliquotiert und durch Einfrieren bei - 85°C bis ± 5°C in ein Tumorzelllysat überführt. Die Qualitätskontrollen umfassen die Prüfungen gemäß der Spezifikation auf Zellzahl, Sterilität und Devitalisierung.

### B) Herstellung der dendritischen-Zellen und der Zusammensetzung zur Tumortherapie

Verwendete Medien:
Medium A: RPMI-Medium + 1 % autologes Plasma
Medium B: Medium A + GM-CSF (800 U/ml) + IL-4 (1000 U/ml)
Medium C: Medium B + TNF-α (1000 U/ml) + Prostaglandin E₂ (1µg/ml)

Die buffycoats aus freigegebenen Blutspenden, aus Leukapheresen oder Vollblut aus dem Blutbeutel werden in Zentrifugenröhrchen überführt und zentrifugiert. Die Interphase enthält die mononukleären Zellen(= buffy coat) und wird von den Erythrozyten (unten) und dem Plasma (oben) abgetrennt. Plasma und mononukleäre Zellen werden auf Lymphoprep® (Nycomed) aufgeschichtet und zentrifugiert. Anschließend werden Plasma und mononukleäre Zellen abpipettiert und erneut zentrifugiert. Das Plasma wird abgenommen und für den Ansatz der Medien verwendet. Restliches Plasma wird bei + 2°C bis + 8°C aufbewahrt, um eventuell zusätzliches Medium A herzustellen. Das Zellsediment wird mit NaCl-Lösung (0,9 %) zweimal gewaschen und zentrifugiert. Vor dem zweiten Waschschritt werden vitale Zellen nach Färbung mit Trypanblau gezählt. Der Zentrifugationsrückstand wird in einer Zellkonzentration von 4x10⁶ /ml in Medium A aufgenommen. Die Zellsuspension wird auf Petrischalen aufgebracht und für zwei Stunden bei 37 °C +/- 1°C/5 % CO₂ inkubiert. Es erfolgt mikroskopische Kontrolle auf adhärente Zellen (Monozyten), wonach Medium A vorsichtig abgesaugt wird, um nicht-adhärente Zellen zu entfernen.

Zu den Petrischalen wird Medium B gegeben und bei 37°C +/- 1°C/5 % CO₂ inkubiert. Am Tag 1 wird Medium B abgesaugt und frisches Medium hinzugefügt. Am Tag 2 wird Medium B partiell (3 ml) abgesaugt und frisches Medium B (3 ml) hinzugefügt. Am Tag 5 erfolgt mikroskopische Kontrolle, ob adhärente Zellen in das nicht-adhärente Stadium übergegangen sind. Die Zellen eines Ansatzes werden vereinigt und vitale Zellen nach Färbung mit Trypanblau gezählt. Die Zellen werden abzentrifugiert und in einer berechneten Menge (5x10⁵ /well/3 ml) in Medium C aufgenommen, wobei das Volumen einem Zehntel des Endvolumens entspricht, mit einer berechneten Menge Tumorzelllysat (5x10⁴ /well/3ml) versetzt, homogenisiert und für eine Stunde bei 37°C +/- 1°C/5 % CO₂ inkubiert, wonach auf das Endvolumen mit Medium C aufgefüllt wird. Die Zellsuspension wird auf 6-well-Platten ausplattiert und weiter bei 37°C +/- 1°C/5 % CO₂ inkubiert. An Tag 6 und 7 erfolgt mikroskopische Kontrolle: der Reifungsprozess der dendritischen Zellen deutet sich durch "Segelbildung" an. Am Tag 8 erfolgt bei ausgeprägter "Segelbildung" bei mikroskopischer Kontrolle das "Ernten" der reifen dendritischen Zellen. Die reifen dendritischen Zellen werden zentrifugiert und zweimal gewaschen. Der Zentrifugationsrückstand wird in 0,9 %iger NaClLösung aufgenommen, vitale Zellen werden nach Färbung mit Trypanblau gezählt und mit 0,9 % NaCILösung auf die gewünschte Zellzahl eingestellt.

## Patentansprüche

1. Zusammensetzung erhältlich durch ein Verfahren, in dem Tumormaterial bonitiert, zerkleinert und in eine gereinigte Zellsuspension überführt wird, die mit Interferongamma und Tocopherolacetat inkubiert und eingefroren wird, wodurch ein Tumorzelllysat entsteht,
und in dem Monozyten aus buffy coats oder aus Vollblut isoliert werden, die anschließend durch Inkubation mit Zytokinen zur Differenzierung zu dendritischen Zellen angeregt und in das nichtadhärente Stadium überführt werden,
wonach eine berechnete Menge des obigen eingefrorenen Tumorzelllysats aufgetaut wird, als Antigen zugesetzt wird, Zytokine zugesetzt werden, inkubiert wird und die entstandenen reifen dendritischen Zellen geerntet werden.

2. Zusammensetzung nach Anspruch 1, bei deren Erzeugung zur Differenzierung zu unreifen dendritischen Zellen IL-4 und GM-CSF zugesetzt werden.

3. Arzneimittel, enthaltend eine Zusammensetzung gemäß mindestens einem der Ansprüche 1-2.

4. Verfahren zur Herstellung eines Arzneimittels, bei dem eine Tumorzellensuspension hergestellt wird, die Tumorzellen abgetötet werden,
und bei dem Monozyten aus Blut isoliert werden, deren Differenzierung zu dendritischen Zellen induziert wird,
und die so gewonnenen unreifen dendritischen Zellen mit dem Zelllysat der abgetöteten Tumorzellen inkubiert werden, die Reifung der dendritischen Zellen induziert wird und die reifen dendritischen Zellen geerntet werden.

5. Verfahren gemäß Anspruch 4, bei dem die Monozyten aus buffy coats, Vollblut, Leukapherese oder Stammzellseparation isoliert werden.

6. Verfahren gemäß Anspruch 4 und/oder 5, bei dem die Differenzierung der Monozyten zu unreifen dendritischen Zellen durch Zytokine, IL-4, und GM-CSF mit oder ohne Interferon-gamma induziert wird.

7. Verfahren gemäß mindestens einem der Ansprüche 4 - 6, bei dem die Reifung von unreifen zu reifen dendritischen Zellen durch Prostaglandin E₂ und TNF-α und/oder mit IL-1β und IL-6 zusätzlich zu IL-4 und GM-CSF induziert wird.

8. Verfahren gemäß mindestens einem der Ansprüche 4 - 7, bei dem die Tumorzellensuspension hergestellt wird, indem Tumormaterial isoliert und gegebenenfalls bonitiert, zerkleinert und in eine gereinigte Zellsuspension überführt wird.

9. Verfahren gemäß mindestens einem der Ansprüche 4 - 8, bei dem die Tumorzellensuspension hergestellt wird, indem autologes Tumormaterial isoliert und gegebenenfalls bonitiert, zerkleinert und in eine gereinigte Zellsuspension überführt wird.

10. Verfahren gemäß mindestens einem der Ansprüche 4 - 9*,* bei dem vor dem Abtöten der Tumorzellen in der Tumorzellensuspension die Expression von membranständigen Proteinkomplexen induziert wird.

11. Verfahren gemäß Anspruch 10, bei dem die Expression von membranständigen Proteinkomplexen durch Interferon-gamma und Tocopherolacetat induziert wird.

12. Verfahren gemäß Anspruch 4, bei dem die Tumorzellen durch Einfrieren abgetötet werden.

13. Verfahren gemäß Ansprüch 4, bei dem die reifen dendritischen Zellen bei Vorliegen typischer morphologischer Merkmale (z. B. Segelbildung), beurteilt durch mikroskopische Kontrolle, und/oder durch Charakterisierung von Oberflächenantigenen mittels fluoreszierenden Antikörpern geerntet werden.

14. Verwendung der Zusammensetzung nach Anspruch 1 zur Herstellung eines Arzneimittels zur Tumortherapie.

15. Verwendung der Zusammensetzung nach Anspruch 1 zur Herstellung eines Arzneimittels zur Tumorimpfung.

## Claims

1. A composition obtainable by a process in which tumor material is evaluated, comminuted and transferred into a purified cell suspension, which is then incubated with interferon-gamma and tocopherol acetate and frozen to form a tumor cell lysate,
and in which monocytes are isolated from buffy coats or whole blood and subsequently induced to differentiation into dendritic cells by incubation with cytokines and converted to the non-adherent stage,
whereupon a calculated amount of the above frozen tumor cell lysate is thawed, added as an antigen, cytokines are added, incubation is performed, and the mature dendritic cells produced are harvested.

2. The composition according to claim 1, wherein IL-4 and GM-CSF are added for differentiation to immature dendritic cells in the preparation.

3. A medicament containing a composition according to at least one of claims 1 and 2.

4. A method for preparing a medicament in which a suspension of tumor cells is prepared, the tumor cells are killed, and monocytes are isolated from blood, their differentiation into dendritic cells is induced,
and the thus obtained immature dendritic cells are incubated with the cell lysate of the killed tumor cells, the maturing of the dendritic cells is induced, and the mature dendritic cells are harvested.

5. The method according to claim 4, in which the monocytes are isolated from buffy coats, whole blood, leukaphereses, or separated stem cells.

6. The method according to claim 4 and/or 5, in which the differentiation of the monocytes into immature dendritic cells is induced by cytokines, IL-4 and GM-CSF with or without interferon-gamma.

7. The method according to at least one of claims 4 to 6, in which the maturing from immature to mature dendritic cells is induced by prostaglandin E₂ and TNF-α and/or IL-1β and IL-6 in addition to IL-4 and GM-CSF.

8. The method according to at least one of claims 4 to 7, in which the tumor cell suspension is prepared by isolating and optionally evaluating tumor material, which is then comminuted and transferred into a purified cell suspension.

9. The method according to at least one of claims 4 to 8, in which the tumor cell suspension is prepared by isolating and optionally evaluating autologous tumor material, which is then comminuted and transferred into a purified cell suspension.

10. The method according to at least one of claims 4 to 9, in which the expression of membrane-borne protein complexes is induced in the tumor cell suspension prior to said killing of the tumor cells.

11. The method according to claim 10, in which the expression of membrane-borne protein complexes is induced by interferon-gamma and tocopherol acetate.

12. The method according to claim 4, in which the tumor cells are killed by freezing.

13. The method according to claim 4, in which the mature dendritic cells are harvested when typical morphological characteristics are present (e.g., veil formation) as evaluated by microscopic check and/or by characterization of surface antigens using fluorescent antibodies.

14. Use of the composition according to claim 1 for preparing a medicament for tumor therapy.

15. Use of the composition according to claim 1 for preparing a medicament for tumor vaccination.

## Revendications

1. Composition pouvant être obtenue par un procédé, dans lequel on contrôle de la matière tumorale, on la broie et on la transforme en une suspension cellulaire purifiée, qu'on incube avec de l'interféron gamma et du tocophérol acétate et qu'on congèle, de manière à obtenir un lysat de cellules tumorales,
et dans lequel on isole des monocytes à partir de couches leucoplaquettaires ou de sang total, qu'on stimule ensuite par incubation avec des cytokines pour différencier des cellules dendritiques et qu'on transforme au stade non adhérent,
après quoi on décongèle une quantité calculée dudit lysat de cellules tumorales congelé, on l'introduit en tant qu'antigène, on ajoute des cytokines, on incube le tout et on collecte les cellules dendritiques matures constituées.

2. Composition selon la revendication 1, pour la préparation de laquelle on ajoute, pour induire une différenciation visant à déterminer des cellules dendritiques immatures, de l'IL-4 et du GM-CSF.

3. Médicament contenant une composition selon au moins l'une des revendications 1 et 2.

4. Procédé de préparation d'un médicament, dans lequel on prépare une suspension de cellules tumorales, on tue les cellules tumorales, et dans lequel on isole des monocytes à partir de sang, pour lesquelles on induit une différenciation visant à déterminer des cellules dendritiques,
et on incube les cellules dendritiques immatures ainsi obtenues avec le lysat cellulaire des cellules tumorales tuées, on induit la maturation des cellules dendritiques et on collecte les cellules dendritiques matures.

5. Procédé selon la revendication 4, dans lequel on isole les monocytes à partir de couches leucoplaquettaires, de sang total, de leucaphérèse ou de séparation de cellules souches.

6. Procédé selon la revendication 4 ou 5, dans lequel on induit la différenciation des monocytes pour déterminer les cellules dendritiques immatures par des cytokines, de l'IL-4 et du GM-CSF avec ou sans interféron-gamma.

7. Procédé selon au moins l'une des revendications 4 à 6, dans lequel on induit la maturation des cellules dendritiques immatures en cellules matures par de la prostaglandine E₂ et du TNF α et/ou avec de l'IL-1β et de l'IL-6 en plus de l'IL-4 et du GM-CSF.

8. Procédé selon au moins l'une des revendications 4 à 7, dans lequel on prépare la suspension de cellules tumorales en isolant de la matière tumorale et éventuellement en la contrôlant, en la broyant et en la transformant en une suspension cellulaire purifiée.

9. Procédé selon au moins l'une des revendications 4 à 8, dans lequel on prépare la suspension de cellules tumorales en isolant de la matière tumorale autologue et éventuellement en la contrôlant, en la broyant et en la transformant en une suspension cellulaire purifiée.

10. Procédé selon au moins l'une des revendications 4 à 9, dans lequel on induit l'expression de complexes de protéines membranaires avant de tuer les cellules tumorales dans la suspension de cellules tumorales.

11. Procédé selon la revendication 10, dans lequel on induit l'expression des complexes de protéines membranaires par de l'interféron-gamma et du tocophérol acétate.

12. Procédé selon la revendication 4, dans lequel on tue les cellules tumorales par congélation.

13. Procédé selon la revendication 4, dans lequel on détermine les cellules dendritiques matures par la présence de caractéristiques morphologiques types (par ex. la formation de voile) au moyen d'un contrôle microscopique, et/ou on les collecte par caractérisation d'antigènes de surface au moyen d'anticorps fluorescents.

14. Utilisation de la composition selon la revendication 1 pour préparer un médicament destiné au traitement des tumeurs.

15. Utilisation de la composition selon la revendication 1 pour préparer un médicament destiné à l'inoculation de tumeurs.
